(19)

Europäisches Patentamt

European Patent Office

Office européen des brevets

(11) **EP 2 500 725 A1**

(12) **EUROPEAN PATENT APPLICATION**

(43) Date of publication:
**19.09.2012 Bulletin 2012/38**

(51) Int Cl.:
*G01N 33/12* $^{(2006.01)}$    *F24C 15/00* $^{(2006.01)}$
*A23L 1/01* $^{(2006.01)}$

(21) Application number: **11158729.1**

(22) Date of filing: **17.03.2011**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(71) Applicant: **Electrolux Home Products Corporation N.V.**<br>**1130 Brussel (BE)** | (72) Inventors:<br>• **Luckhardt, Christoph**<br>  **91607 Gebsattel (DE)**<br>• **Ruther, Florian**<br>  **91628 Steinsfeld (DE)**<br><br>(74) Representative: **Schröer, Gernot H.**<br>**Meissner, Bolte & Partner GbR**<br>**Bankgasse 3**<br>**90402 Nürnberg (DE)** |

(54) **A method for controlling a cooking process of food stuff**

(57)    A method for controlling a cooking process of food stuff (20) comprising the steps of:

a) detecting at least one physical property, in particular an electrical impedance (Z) or at least one component thereof and/or a temperature (T), at at least one check point or at a plurality of check points (22) in a, preferably inner, region of the food stuff (20),

b) deriving from said at least one detected physical property, in particular impedance or component thereof and/or temperature, food type information about the type of food of the food stuff (20) and/or food age information about the age of the food stuff (20),

c) deriving from said at least one detected physical property, in particular impedance or component thereof and/or temperature, a bacterial information (e.g. F or F < $F_0$) about the bacterial concentration in the food stuff (20) or about a reduction in the bacterial concentration in the food stuff (20),

d) using the food type information of the food stuff (20) and/or the food age information of the food stuff (20) in said step of deriving the bacterial information (e.g. F or F < $F_0$),

e) providing said bacterial information (e.g. F or F < $F_0$) to a user interface and/or to an information output unit and/or to a control unit for adapting the cooking time and/or cooking temperature dependent on this bacterial information.

FIG 9

**Description**

The present invention relates to a method for controlling a cooking process of food stuff. Further, the present invention relates to a food probe for use in such a method.

The cooking process of substantially homogenous food stuffs like meat, potatoes and other vegetables, pies and some casseroles is monitored mainly by detecting the core temperature of the food stuff. The user has to know at which core temperatures the food stuff has certain desired properties like colour, tenderness or degree of cooking.

In order to simplify the cooking process for the user, automatic cooking functions are available. Said cooking functions are based on an information input from the user and/or measurements by sensors such as temperature probes. The determination of food properties by measurements allows a reduction of the information input from the user.

The detection of the electrical impedance of the food stuff can provide a lot of information for the automatic cooking functions.

US 2006/0174775 A1 discloses a method and an apparatus for tracing a cooking process. At least two separated electrodes are inserted into the food stuff. The electrical impedance is measure at a certain frequency in order to monitor the cooking process.

DE 36 21 999 A1 discloses a method for monitoring a cooking process of food. An elongated food probe with at least two electrodes in the front portion is inserted into the food stuff. An electrical impedance of the food stuff is detected in the beginning of the cooking process and during the further cooking process.

EP 1 317 643 B9 discloses a method for running a food cooking oven, wherein an elongated pin food probe is introduced in the interior of the food stuff. The food probe comprises a plurality of temperature sensors located at different points of said food probe. A value F that is representative of the reduction in the bacterial content of the cooked food and depends on the detected temperature and the cooking time is calculated by a control device. For this calculation of reduction in bacterial content the lowest temperature detected by the plurality of temperature sensors of the food probe is selected. Furthermore selector means are provided by which the user enters information about the food type or category and dependent on the selected food category different and a multiplicity $F_0$, $F_1$ ,.. $F_N$ of pre-defined threshold values depending on the selected food category is sent to the control device for comparison with the actual calculated value whether $F > F_i$ for i= 0, 1, .. n wherein a safety condition is $F > F_0$. Thereby, an evolution pattern of the reduction of the amount of bacteria can be plotted by checking when the next threshold-value $F_{i+1}$ is reached by the value F. The final factor of reduction of bacteria is then n = $F/F_0$.

However, in none of these known systems the type of food stuff can be recognized automatically.

It is an object of the present invention to provide a new method for controlling a cooking process of food stuff.

This object is achieved by the method having the features of claim 1. Further embodiments and improvements can be obtained from the dependent claims.

The method according to claim 1 is provided for controlling (or: running) a cooking process of food stuff, in particular in a cooking oven or cooking vessel, and comprises the steps of:

a) detecting at least one physical property (or: quantity), in particular an electrical impedance or at least one component thereof and/or a temperature, at at least one check location or point (or: measuring point, detection point) or at a plurality of check points in a, preferably inner, region of the food stuff,

b) deriving from said at least one detected physical property, in particular impedance or component thereof and/or temperature, at least one of food type information about the type of food of the food stuff and food age information about the age of the food stuff,

c) deriving from said at least one detected physical property, in particular impedance or component thereof and/or temperature, a bacterial information about the bacterial concentration in the food stuff or about a reduction in the bacterial concentration in the food stuff,

d) using either the food type information of the food stuff or the food age information of the food stuff or both, food type information and food age information, in said step of deriving the bacterial information,

e) providing said bacterial information to a user interface and/or to an information output unit and/or to a control unit for adapting the cooking time and/or cooking temperature dependent on this bacterial information.

It is understood that said steps of deriving information in features b) and c) can be performed in arbitrary order.

Furthermore, the term bacterial information is to be interpreted in a broad sense including any information, such as values, signals, indications, symbols, words or the like, about the bacterial concentration or a reduction thereof directly or information derived or evaluated therefrom. In particular, the term bacterial information also includes information derived from a comparison of a value or signal representing the bacterial concentration or a reduction thereof with at least one pre-given reference value or reference signal, for instance an information about whether the bacterial concentration or a reduction thereof is within a specified safety range or below or above a certain specified safety level, or any other further evaluation of a value or signal for the bacterial concentration or a reduction thereof.

In a preferred embodiment at least one or a plurality of electric voltage(s)(or: electric field(s)) in general having AC components or being an AC voltage is applied to said region of the food stuff and the impedance or the at least one component thereof in that region of the food stuff is measured or detected. Preferably at least one food probe is used which is inserted into the food stuff and comprises at least two electrodes or a plurality of electrode pairs for applying the electric voltage and measuring the impedance or the at least one component thereof. Alternatively, a number of food probes or food probe bodies is used which are inserted into the food stuff and comprises at least two electrodes in each case for applying the electric voltage and measuring the impedance or the at least one component thereof.

The impedance can for instance be measured or determined by measuring the electric voltage and the electric current in the region of the food stuff, in particular between the electrodes, which gives information on the impedance, including the phase angle between voltage and current. Of course measuring the impedance also includes using the measured values of the voltage and current directly, as they are unambiguously related to the impedance, without an intermediate calculation of the impedance.

The at least one electrical component of the electrical impedance is in particular chosen from the group comprising the ohmic resistance, the reactance, the capacity, the inductivity, the modulus and the phase angle.

During the step of deriving food age information about the age or freshness of the food stuff from said detected at least one physical property, in particular impedance or component thereof, in one variant according to the invention a comparison is made with stored pre-determined or empirically determined reference data for age or freshness of different food types, including in particular different types of meat and/or fish, in particular stored in a reference data base and/or as reference data sets.

In a further preferred embodiment information about the food type of the food stuff, including in particular different types of meat and/or fish and/or vegetables, is derived from said detected values of the at least one physical property, in particular impedance or component thereof.

Also during this deriving of information about the food type from said at least one physical property, in particular detected impedance or component thereof, a comparison can be made with stored pre-determined or empirically determined reference data for different food types, including in particular different types of meat and/or fish and/or vegetables, in particular stored in a reference data base and/or as reference data sets.

This deriving or determining of the food type is preferably made or performed before the deriving of information about the age or freshness of the food stuff and/or preferably based on the same detected impedance or component values as with the deriving of food age information about the age or freshness of the food stuff. This means that the detection or measurement of at least one physical property, in particular impedance or component thereof, has to be made only once and then firstly the food type and then with the information on the food type, the age or freshness of the food stuff of this food type is determined, wherein only a set of reference data for only this food type has to be accessed. But it is also possible to make separate measurements for type of food stuff and age of foodstuff or other characteristic property respectively.

The reference data supplied by the manufacturer can be supplemented during use by the user who can add other food types or food age criteria through an input device.

In a preferred embodiment the at least one physical property, in particular electrical impedance or the at least one component thereof, of the food stuff for deriving of information on the age or freshness or type of the food stuff is detected before or at the beginning of the cooking process and/or at a predefined, preferably low, temperature e.g. room temperature, or in a predefined temperature range, preferably low, temperature e.g. room temperature. This way the influence of temperature on the measurements is greatly reduced.

Furthermore the at least one physical property, in particular electrical impedance or the at least one component thereof, of the food stuff for a subsequent deriving of information on the age or freshness or type of the food stuff can be measured several times in a pre-given time interval, e.g. several minutes, at pre-determined instants of time, e.g. every 10 to 60 seconds, to reduce measuring errors or get additional information about the food stuff.

In a further advantageous embodiment the food age information of the food stuff is used for controlling or adapting the cooking time and/or cooking temperature of the cooking process dependent on this information about the age or freshness. For instance, for less fresh or aged food stuff the cooking time is selected to be longer and/or the cooking temperature is selected to be higher or to be at a high level for a longer time.

The detection or measurement of the electrical impedance or the at least one component thereof can be made at a single frequency, in particular of the electric field, which in most cases allows for a sufficient distinction between the different food types or food characteristics or properties already.

However, the deriving or recognition or determining of the information on the food type or food characteristics can be even improved if the measurement or detection is made at two or even more frequencies, in particular of the electric field applied, which allows for an even clearer and in all case unambiguous recognition of food type and food age.

When two different frequencies are used, two reasonable frequencies are 50 kHz and 5 kHz.

The values of the impedance or component thereof detected at the one, two or more frequencies are in a preferred embodiment compared with the same number of stored reference values for food types and/or age or freshness of food stuff previously determined at the same frequencies.

The food type or food stuff age is derived in particular by determining the highest degree of coincidence with the reference values, e.g. by some mathematical norm, for instance Euclidian norm, or metric, or by using a function, e.g. fit function, of the detected values of the impedance or component thereof at the two or more frequencies.

In other variants, the information on the age or freshness or type of the food stuff can be derived from a ratio or a difference or sum or even a ratio of a difference and the sum of two detected values of the impedance or component thereof at two different frequencies.

In one embodiment the ohmic resistance is used as the component of the impedance to derive information on type or age of the food stuff, as the ohmic resistance differs over a same frequency spectrum for different food types as well as for fresh food stuff as compared to aged food stuff in absolute values as well as in the first derivative and the second derivative. In particular, at lower frequencies, the ohmic resistance of fresh food stuff, in particular meat such as poultry, is higher than that of aged food stuff. Further the ohmic resistance of fresh food stuff decreases more steeply with increasing frequency than that of aged food stuff. Also, at high frequencies, the ohmic resistance of the fresh food stuff is higher than that of aged food stuff. It can even be evaluated that the curve of the ohmic resistance for fresh food stuff can have a turning point in contrast to aged food stuff. The used value of the ohmic resistance may be selected from those values of the ohmic resistance at the different check points. For example, one of the usual values is selected and the outliers are ignored. Alternatively, the used value of the ohmic resistance is the mean value calculated from those values of the ohmic resistance at the different check points.

In an alternative embodiment the reactance is used as the component of the impedance to derive information on type or age of the food stuff, as also the reactance differs over a same frequency spectrum for different food types as well as for fresh food stuff as compared to aged food stuff in absolute values as well as in the first derivative and the second derivative. For instance, the reactance of fresh food stuff, in particular meat such as poultry, as well as of aged food stuff has a minimum in a given frequency spectrum and the reactance of the fresh food stuff at the minimum is smaller than the reactance (X) of the aged food stuff at the minimum. The used value of the reactance may be selected from those values of the reactance at the different check points. One of the typical values may be selected and the outliers may be ignored. Further, the used value of the reactance may be the mean value calculated from those values of the reactance at the different check points.

In yet another embodiment the phase angle is used as the component of the impedance to derive information on type or age of the food stuff, as also the phase angle differs over a same frequency spectrum for different food types as well as for fresh food stuff as compared to aged food stuff in absolute values as well as in the first derivative and the second derivative. For instance, the phase angle of fresh food stuff, in particular meat such as poultry, has a minimum in a given frequency spectrum whereas the phase angle of aged food stuff has no minimum in this given frequency spectrum. Also the ratio of phase angles at two different frequencies decreases with increasing age of the food stuff and is in particular used to check the quality of the food stuff before or at the beginning of the cooking process. In particular, the used value of the phase angle and/or the ratio of phase angles is selected from those values of the phase angles and/or the ratio of phase angles, respectively, at the different check points. Preferably, one of the usual values is selected and the outliers are ignored. Alternatively, the used value of the phase angle and/or the ratio of phase angles are the mean value calculated from those values of the phase angle and/or the ratio of phase angles, respectively, at the different check points.

The deriving from said detected impedance or component thereof information about the age or freshness of the food stuff can be performed in particular by calculating one or more electrical parameters of the food stuff from the electrical impedance or component thereof and comparing said electrical parameters with a data base. In particular, the used value of the ratio of phase angles corresponds with the temperature at the coldest check point of the food stuff.

Furthermore, in a specific embodiment, the electrical impedance or the at least one component thereof of the food stuff is detected again in another step during the cooking process and/or when the food stuff has been subjected to cooking already, for determining the cooking progress and/or cooking temperature in the region of the food stuff. In particular a ratio of phase angles at two different frequencies is calculated as a function of the temperature of the food stuff.

Further, the present invention relates to a food probe for inserting into food stuff, being provided for use in the method according to the invention and comprising according to claim 14:

- at least one probe body, in particular an elongated rod and/or in particular made of a non-conductive material,
- wherein at least a probing portion of the probe body is provided for inserting into the food stuff,
- at least one pair of electrodes arranged at the probing portion of the probe body, in particular a plurality of pairs of electrodes, preferably arranged serially or one after the other along a longitudinal axis of the probe body,
- wherein each pair of electrodes comprises a first electrode and a second electrode arranged in a predetermined distance from each other, and is connected or connectable to a voltage supply for applying a voltage between the first electrode and the corresponding second electrode and to a control unit of a cooking oven or cooking appliance for measurement of the physical property, in particular impedance or component thereof, of the food stuff (arranged in between the electrodes,

- wherein for at least one, preferably each, check point in the food stuff at least a pair of electrodes is provided.

In a specific embodiment the food probe comprises at least one temperature sensor arranged at the probe body, wherein for or at least one, preferably each, check point in the food stuff at least one temperature sensor is provided for measuring the temperature at that check point.

This one food probe with several electrodes and/or temperature sensors allows measurements at several check points inside the food stuff. The user has to insert only one food probe into the food stuff and obtains a plurality of check points inside the food stuff. But of course also a set of food probes can be provided each having at least one but less than the overall number of electrodes or temperature sensors.

The user can individually select the check points at the food stuff.

Also the method and food probe according to the invention can in all embodiments be combined with temperature measurements using separate temperature sensors to obtain further information about the food stuff and cooking process. For instance during the deriving of the bacterial information the temperature at each check point can be detected, in particular with a corresponding temperature sensor, and the lowest temperature at all check points be uses for deriving the bacterial information, as for instance described in EP 1 317 643 B9.

At last, the food probes described above can be commonly used for the method described above.

A preferred application of the method and food probe according to the invention is for use in foodservice or catering.

The invention will be described in further detail with reference to the drawings, in which

FIG 1 illustrates a schematic view of a food probe according to a preferred embodiment of the present invention,

FIG 2 illustrates a schematic diagram of a phase angle as a function of the frequency for several types of food according to the preferred embodiment of the invention,

FIG 3 illustrates a schematic diagram of a ratio of two phase angles at different frequencies as a function of the temperature according to the preferred embodiment of the invention,

FIG 4 illustrates a schematic diagram of an ohmic resistance as a function of the frequency for poultry of different age according to the preferred embodiment of the present invention,

FIG 5 illustrates a schematic diagram of a reactance as a function of the frequency for poultry of different age according to the preferred embodiment of the present invention,

FIG 6 illustrates a schematic diagram of a phase angle as a function of the frequency for poultry of different ages according to the preferred embodiment of the present invention,

FIG 7 illustrates a schematic diagram of the ratio of two phase angles at different frequencies as a function of the age of the food stuff according to the preferred embodiment of the present invention,

FIG 8 illustrates a schematic diagram of a number of bacteria as a function of the age of the food stuff according to the preferred embodiment of the present invention, and

FIG 9 illustrates a schematic diagram of a temperature and a sterilizing effect of the food stuff as a function of the time according to the preferred embodiment of the invention.

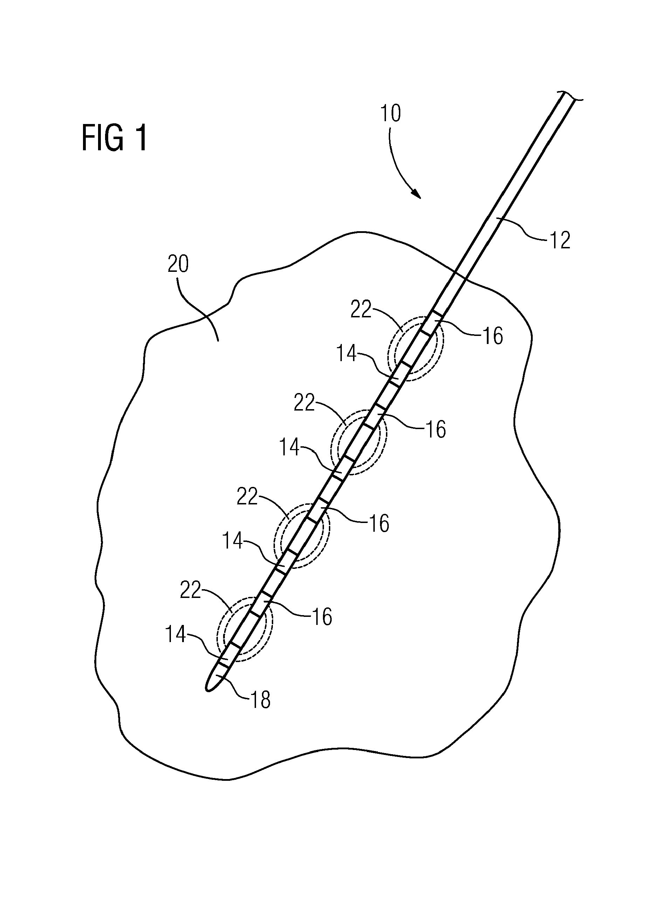

FIG 1 illustrates a schematic view of a food probe 10 according to a preferred embodiment of the present invention. The food probe 10 is provided for recognizing the type and/or characteristics of food stuff and monitoring a cooking process of such food stuff in a cooking oven or a cooking vessel.

The food probe 10 comprises an elongated rod 12, a plurality of pairs of electrodes and a spike 18. Each pair of electrodes includes a first electrode 14 and a second electrode 16. The first electrodes 14 and the second electrode 16 are arranged alternatingly along the rod 12. In this example, the food probe 10 comprises four pairs of electrodes. A front portion of the food probe 10 is penetrated or inserted into food stuff 20. The food probe 10 should be penetrated or inserted into the food stuff 20, so that as much as possible of the electrodes 14 and 16 are inside the food stuff 20.

The rod 12 is made of a non-conductive material. The first electrodes 14 and the second electrodes 16 are made of a conductive material. The spike 18 is made of a non-conductive material again.

The spike 18 may be made of the same non-conductive material as the rod 12 or by another non-conductive material. Alternatively, the spike 18 may be made of a conductive material.

The spike 18 is arranged at a front end of the rod 10. The spike 18 allows that the food probe 10 can easily be inserted or penetrated into the food stuff 20.

The first electrodes 14 and the second electrodes 16 are arranged in the front and central portions of the rod 12. One of the first electrodes 14 is arranged besides the spike 18. The corresponding second electrode 16 is arranged in a predetermined distance from said first electrode 14. All electrodes 14 and 16 are electrically isolated from each other.

When the front portion of the food probe 10 is inside the food stuff 20 in an inner region thereof, then the first electrodes 14 and the second electrodes 16 are also arranged inside the food stuff 20.

When voltages are applied between the first electrodes 14 and the second electrodes 16 in each case, electric fields or voltages 22 are generated or present within the food stuff 20 in its inner region. Each electric voltage or field 22 extends between and in the environment of the first electrode 14 and the second electrode 16 of the corresponding pair of electrodes and usually at least contains AC components or is a AC voltage.

Preferably, the front portion of the food probe 10 is invaded into the food stuff 20 in such a way, that at least one of the electric fields 22 is generated within the central portion of the food stuff 20.

The serially arranged electrodes at the food probe 10 allow the generation of the electrical fields 22 in the core or a central inner region of the food stuff 20. The food probe 10 is a compact tool and easy to handle.

The food probe 10 is provided for probing the food stuff 20 and in particular for detecting an electrical impedance Z of the food stuff 20 in an inner region thereof by applying the electric field 22 between the corresponding electrodes 14 and 16 in this region and measuring the impedance Z or an electrical component thereof between the electrodes 14 and 16.

The food probe 10 allows the measurement of the electrical impedance Z at several checkpoints inside the food stuff 20 at the same time or at different times. Each checkpoint corresponds with one pair of the electrodes 14 and 16.

According to an alternative embodiment of the present invention a plurality of food probes 10 are provided. Said food probes 10 can be invaded into different points of the food stuff 20. In this case, each of said food probe 10 requires only one pair of electrodes 14 and 16. Preferably, the both electrodes 14 and 16 are arranged in the front portion of the food probe 10. However, a plurality of food probes 10 may be provided, wherein one or more of said food probes 10 include several pairs of electrodes 14 and 16.

The impedance Z or an electrical component thereof can for instance be measured or determined by measuring the electric voltage 22 and the electric current in the region of the food stuff, in particular between the electrodes and/or by means of LCR metering, and using basically the complex number relation between complex voltage U and complex current I with the complex impedance Z:

$$U(t) = Z(t) * I(t)$$

So, in all embodiments, when the detecting of impedance Z and components thereof and information derived thereof is mentioned this of course also includes using the measured values of voltage and current directly as these are measured values representing the impedance and components thereof or being unambiguously related thereto.

An evaluation unit, which is in particular part of or integrated in a control unit, for instance a microprocessor with corresponding storage (none shown), now evaluates the influence the food stuff 20 has on this measured impedance Z, in particular at two or more frequencies, and preferably measured in the beginning of the cooking process and during the further cooking process. The evaluation or control unit is electrically connected with all electrodes 14 and 16 of the food probe 10 and provides the electrical voltage for the measurement and measures the (drop of the) voltage and/or the current over time or, alternatively, frequency between the electrodes 14 and 16.

In particular, the detected values of the electrical impedance Z may be compared with a reference data base, which is integrated in or accessed by the evaluation or control unit, in order to determine the type and/or characteristics of food. This will be explained in more detail in the following.

The electrical impedance Z is the electrical resistance for DC and/or AC currents and mathematically it is a complex number which can be represented by

$$Z = Re(Z) + i*Im(Z) = R + i*X \qquad\qquad (1)$$

including the imaginary unit i.

The ohmic resistance (or: DC resistance) R is the real part Re(Z) of the electrical impedance Z.

The reactance (or: AC resistance) X is the imaginary part Im(Z) of the electrical impedance Z. The reactance X can, for example, be purely capacitive and is then $X = -1/(2\pi*f*C)$ with the frequency f of the electric voltage and with the electrical capacitance C. The reactance X can also, for example, be purely inductive and is then $X = +2\pi*f*L$ with the frequency f and the inductance L.

The representation of the electrical impedance Z by polar coordinates

$$Z = Mod(Z) * exp(i*\phi) \qquad\qquad (2)$$

includes the modulus Mod(Z) and the phase angle $\phi$ of said electrical impedance Z.

Further, the electrical impedance Z can be represented by the cosine and sine functions of the phase angle $\phi$:

$$Z = Mod(Z) * cos\ \phi + i * Mod(Z) * sin\ \phi. \qquad (3)$$

as $exp(i*\phi) = cos\ \phi + i * sin\ \phi$.

In particular the values of the cosine and sine functions of the phase angle $\phi$ are also parameters, which correlate with the type and properties of the food stuff. The determining or calculation of the cosine and/or sine of the phase angle $\phi$ from the electrical impedance Z provides further characteristic parameters of the food stuff.

Beside the cosine and sine functions, also the tangent and cotangent functions of the phase angle $\phi$ can be calculated from the electrical impedance Z. The trigonometric functions of the phase angle $\phi$ are suitable to provide other correlations with the properties of the food stuff as the phase angle $\phi$ itself, since each value of the trigonometric function corresponds with two values of the phase angle $\phi$.

Therefore, whenever in this application it is referred to an impedance Z to be detected or determined this implies that also one of the aforementioned components R, X, C, L, Mod(Z), $\phi$, sin $\phi$, cos $\phi$, etc. of the impedance Z alone or any combination thereof can be detected or determined as well.

According to the invention, correlations are used between measured values for or being dependent on the impedance Z itself or at least one of the ohmic resistance R, the reactance X, the modulus Mod(Z) and the phase angle $\phi$ or trigonometric functions thereof of the impedance Z for n=1 one single frequency or preferably n > 1 different frequencies f on the one hand and the types and/or properties of the food stuff on the other hand.

The values of the impedance Z or a component thereof at the n frequencies can be compared or correlated in different ways to determine the type or a property of the food stuff 20.

In a preferred embodiment two values (n = 2) at two different frequencies f1 and f2 are used, e.g. Z1 = Z(f1) or $\phi$1 = $\phi$(f1) and Z2 = Z(f2) or $\phi$2 = $\phi$(f2).

But it is also possible to use only one frequency (n = 1) and the values of the impedance Z or its component at this single frequency.

Alternatively, more than just two frequencies (n > 2), even a high number n of measuring frequencies of several hundred or even thousand can be used if a very detailed analysis with a high resolution is desired, for instance a detailed curve discussion or comparison of curve parameters of curves in a certain frequency spectrum.

Also it is possible to measure the impedance or component thereof several times in a given time interval, for instance every 30 seconds in the first 5 minutes of the cooking process, to average the measurements and decrease measuring inaccuracies.

Now, for instance, in order to determine a type or property, such as freshness, of the food stuff 20, a ratio of two values at the two different frequencies f1 and f2, e.g. Z1/Z2 or $\phi$1/$\phi$2, can be used or a difference of two values at two different frequencies f1 and f2, e.g. Z1 - Z2 or $\phi$1 - $\phi$2, or a ratio of a difference of two values at two different frequencies f1 and f2 and the sum of these two values at two different frequencies, e.g. (Z1 - Z2)/(Z1 + Z2) or ($\phi$1 - $\phi$2)/($\phi$1 + $\phi$2).

Also another function apart from the described ratios or differences of the n values obtained for the n frequencies for n = 1 or n > 1 can be used to calculate parameters representing information on the food stuff from the detected values of the impedance or components thereof.

But it also possible to compare the one, two (or more) values of the impedance Z or its component at the corresponding frequency or frequencies with the same number of previously determined and stored reference values or reference data for different food types at the same frequency or frequencies and to decide where there is the highest degree of coincidence with the reference data of one of the stored food types by some kind of mathematical norm, for instance Euklidian norm, or metric (distance function).

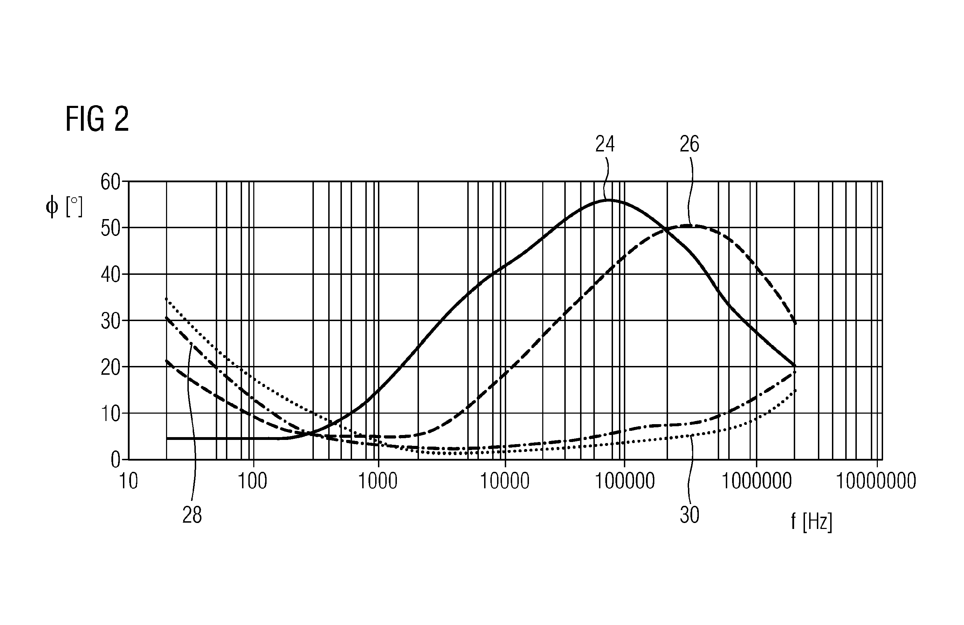

According to the invention it could be shown that different types of food or food stuff 20 have different and in each case characteristic impedances Z or components thereof at the same frequency or frequencies. Examples are shown in FIG 2.

FIG 2 illustrates in a schematic diagram typical functional graphs or curves of the value pairs (f, $\phi$(f)) of the detected or calculated phase angle $\phi$ of the impedance Z as a function of the frequency f in a given frequency spectrum for several types of food according to a preferred embodiment of the invention. These curves or graphs or sets of values are usually empirically determined by calibration or reference measurements using the food probe according to FIG 1 for probing different types of food stuff 20 and storing the data as reference data for the cooking process in the cooking oven or appliance.

The four curves 24, 26, 28 and 30 in FIG 2 show the phase angle $\phi$ over the same frequency spectrum for different types of food stuff 20. A first curve designated by 24 corresponds to potatoes as food stuff 20, a second curve 26 refers to cauliflower, a third curve 28 was determined for pork and a fourth curve 30 for the breast of a turkey hen. The frequency spectrum of the frequency f in FIG 2 extends from about 10 Hz to about 1 MHz.

FIG 2 clarifies that different types of food have their own characteristic phase angle $\phi$ as function of the frequency f and their respective curves 24, 26, 28 and 30 differ significantly in absolute values as well as shape of the curves. The vegetable curves 24 for potatoes and 26 for cauliflower both have a well defined maximum or peak but at different frequency as well as different value of the phase angle. The meat curves 28 and 30 both have a minimum in the middle and less variation in phase angle than the vegetable curves 24 and 26 and differ from each other in the absolute values of the phase angle at same frequencies (except for one single frequency where the two curves 28 and 30 intersect).

Therefore, by taking at least two values $\phi1 = \phi(f1)$ and $\phi2 = \phi(f2)$ of the phase angle $\phi$ at two different frequencies in each curve shown in FIG 2 the curve and thus the corresponding food stuff 20 can be unambigously determined or identified.

The phase angle $\phi$ in a specific frequency spectrum containing at least two frequency values can be detected for an actual food stuff 20 to be cooked, in particular by the food probe 10 and its associated evaluation and/or control unit, and then compared with a data base where calibration or reference curves, like the curves 24, 26, 28, 30, or look-up tables or the like are stored. Thus, the type of food can be automatically recognized. This applies to or is possible at different stages of the cooking process or different temperatures of the various food stuff 20.

Since the one or more food probes 10 includes several check points, the electrical impedance Z and the resulting phase angle $\phi$ are detected at several points inside the food stuff 20. Preferably, a mean value of the detected phase angles $\phi$ or a typical value of the detected phase angles $\phi$ is used for the determination of the kind of food stuff 20. If the food stuff 20 has an inhomogeneous structure, then one or more check points could be in a position, in which the food stuff 20 has untypical properties. Thus, the several check points increase the reliability of the determination of the type of food stuff 20.

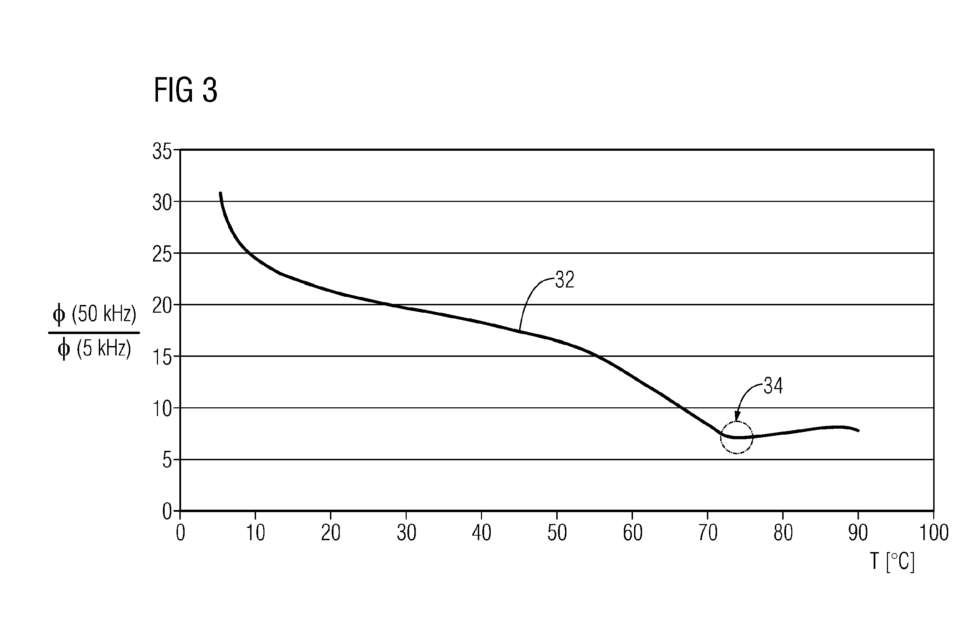

FIG 3 illustrates a schematic diagram of a ratio of two phase angles $\phi$ at different frequencies as a function of the temperature according to the preferred embodiment of the invention. The curve 32 relates to the ratio of the phase angles $\phi$ at the frequencies of 50 kHz and 5 kHz and relates to meat.

The interval of the temperature in FIG 3 extends from about 10 °C to about 90 °C. At a point 34 the curve 32 has a minimum. At this point 34 basically all proteins of the food stuff are denatured, i.e. the meat is fully cooked. The point 34 corresponds with a temperature between 70 °C and 80 °C.

Since the one or more food probes 10 includes several check points, the temperature T is detected at several point inside the food stuff 20. Preferably, the lowest detected value of the temperature T is used for monitoring and controlling the cooking process. In food stuff 20 with a homogeneous structure the coldest point is normally in the centre of the food stuff 20. However, if the food stuff 20 has a heterogeneous structure, then the coldest point may be out of the centre of the food stuff 20. Thus, the plurality of checkpoints allows the determination of the coldest point inside the food stuff 20.

This example in FIG 3 shows that an electrical parameter or variable derived from the impedance Z such as in this case the ratio of the phase angles at two different frequencies can also be used to determine the temperature or to control the cooking process in time in particular by determining the degree or progress of cooking without actually having to measure the temperature directly.

In general it can be said that the impedance Z of the food stuff depends on the degree of destruction in the cells of the food stuff by the cooking process.

So also by these measurements at further stages of the cooking process it is possible to determine or further specify the type of food as different types of food as well as also food of different age show different degradation or denaturation during the cooking process.

After having determined the type of the food stuff 20 which is probed by the food probe 10 or as an alternative or in addition to such determination of the food type according to the invention it is also possible, in a further embodiment according to the invention, to determine also properties or characteristics of the food stuff such as its age by using the impedance or at least one component thereof. The age of the food stuff 20, in particular meat such as poultry or pork or veal or fish etc., can then be used by the control unit of the cooking appliance or oven in order to control the cooking process accordingly, in particular to control the cooking time and/or cooking temperature. Aged food stuff, in particular meat or other food obtained from animals, will usually contain more microorganisms, in particular bacteria, and will thus need more time or higher temperature to sterilize and kill the microorganisms sufficiently. An example of such determination of the age of food stuff will be given in the following.

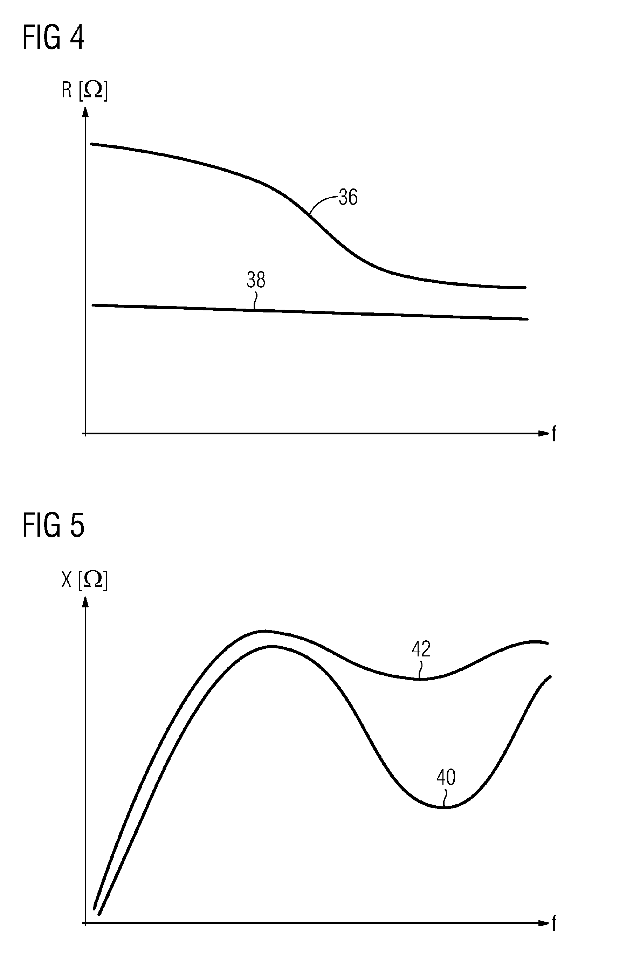

FIG 4 illustrates a schematic diagram of the ohmic resistance R measured for poultry of different age as a function of the frequency f according to the preferred embodiment of the present invention. The ohmic resistance R is the real part of the complex electrical impedance Z. The electrical impedance Z is detected and the ohmic resistance R is then calculated from said detected electrical impedance Z or the ohmic resistance R can also be detected or measured directly.

A typical curve or functional graph of the pairs (f, R(f)) of the value of the detected or calculated ohmic resistance R as a function of the frequency f of fresh poultry is designated by 36 and a corresponding graph or curve of aged poultry by 38. At low frequencies f, the ohmic resistance R of the fresh poultry according to curve 36 is much higher than the ohmic resistance R of aged poultry according to curve 38. The ohmic resistance R of the fresh poultry according to curve 36 decreases clearly with the increasing frequency f and then approaches a roughly constant value again. The ohmic resistance R of the aged poultry according to curve 38 however decreases much less or only marginally with the increasing frequency f and remains almost constant. At high frequencies f, the ohmic resistance R of the fresh poultry according to curve 36 is still a bit higher than the ohmic resistance R of aged poultry according to curve 38.

Thus, the dependency or correlation of the ohmic resistance R of the electrical impedance Z over the same frequency spectrum or interval shown in FIG 4 differs significantly for the fresh poultry according to curve 36 and the aged poultry according to curve 38 or, in other words the functions R(f) for fresh poultry and less fresh or aged poultry are different in absolute values as well as in the first derivative dR/df and the second derivative $d^2R/d^2f$ for instance. Also the curve 36 for fresh poultry may have a turning point or inflection point in contrast to curve 38 for aged poultry.

The electrical impedance Z is detected at several check points inside the food stuff 20. Preferably, a mean value or typical value of the detected electrical impedances Z or the calculated ohmic resistances R is used for the determination of the age or freshness of the food stuff 20. If the food stuff 20 has an inhomogeneous structure, then one or more check points could be in a position, in which the food stuff 20 has untypical properties. Thus, the several check points increase the reliability of the determination of the age or freshness of the food stuff 20.

FIG 5 shows in a schematic diagram the reactance X as a function of the frequency f for poultry of different age according to the preferred embodiment of the present invention. The reactance X is the imaginary part of the complex electrical impedance Z. The electrical impedance Z is detected and the reactance X is then calculated from said detected electrical impedance Z or, again, the reactance X is measured directly.

The curve designated by 40 shows the reactance X of fresh poultry and the curve 42 the reactance X of aged poultry each as a function of the frequency f. At low frequencies f, the reactance X(f) of the fresh poultry according to curve 40 as well as the reactance X of the aged poultry according to curve 42 are small and rise with the increasing frequency f. The reactance X of the fresh poultry in curve 40 has two maxima and a minimum between said maxima. The reactance X of the aged poultry in curve 42 has also two maxima and a minimum at the same frequency ranges. A first maximum is in the central frequency range, and a second maximum is at high frequencies f.

The minima of the reactance X are the most significant difference between the fresh poultry according to curve 40 and the aged poultry according to curve 42. The reactance X of the aged poultry according to curve 42 at the minimum is only marginally smaller than the reactance X of the aged poultry according to curve 42 at the maxima. However, the reactance X of the fresh poultry according to curve 40 at the minimum is substantially lower than the reactance X of the fresh poultry according to curve 40 at the maxima. Further, the reactance X of the fresh poultry according to curve 40 at the minimum is clearly smaller than the reactance X of the aged poultry according to curve 42 at the minimum.

Thus, the curves for the reactance X of the electrical impedance Z over the same frequency spectrum differ significantly for the fresh poultry (curve 40) and the aged poultry (curve 42). in other words the functions X(f) for fresh poultry and aged poultry are different in absolute values as well as in the first derivative dR/df and the second derivative $d^2R/d^2f$ at same frequencies for instance.

Preferably, a mean value or a typical value of the detected electrical impedances Z or the calculated reactance X is used for the determination of the age or freshness of the food stuff 20. If the food stuff 20 has an inhomogeneous structure, then one or more check points could be in a position, in which the food stuff 20 has untypical properties. Thus, the several check points increase the reliability of the determination of the age or freshness of the food stuff 20.

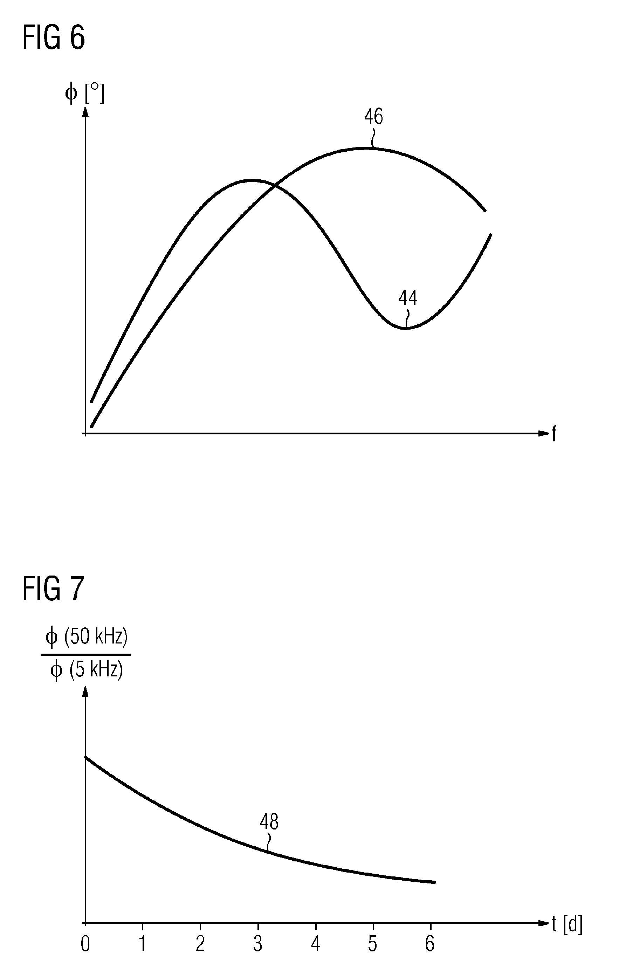

FIG 6 illustrates a schematic diagram of the phase angle $\phi$ as a function of the frequency f for poultry of different age according to the preferred embodiment of the present invention. The phase angle ϕ is the phase part of the complex electrical impedance Z. The electrical impedance Z is detected and the phase angle ϕ is then calculated from said detected electrical impedance Z or the phase angle ϕ is detected directly.

As can be seen in FIG 6, at low frequencies f, the phase angle ϕ of the fresh poultry depicted in curve 44 is small and rises with the increasing frequency f. Two maxima occur in the central frequency range and at high frequencies f in curve 44 for fresh poultry, and there is a clear minimum between the both maxima. In contrast, the phase angle ϕ of the aged poultry in curve 46 has only one maximum and no minima. Thus, the phase angle ϕ of the electrical impedance Z differs significantly for the fresh poultry according to curve 44 and the aged poultry according to curve 46 in the same frequency spectrum.

Preferably, a mean value or a typical value of the detected electrical impedances Z or the calculated phase angle ϕ is used for the determination of the age or freshness of the food stuff 20. If the food stuff 20 has an inhomogeneous structure, then one or more check points could be in a position, in which the food stuff 20 has untypical properties. Thus, the several check points increase the reliability of the determination of the age or freshness of the food stuff 20.

In FIG 7 the ratio of two phase angles ϕ at different frequencies f is depicted as a function of the age t in days (d) of the food stuff according to the preferred embodiment of the present invention. In this example, the frequencies are 50 kHz and 5 kHz. The curve 48 relates to poultry.

FIG 7 shows that the ratio of the two phase angles ϕ decreases with an increasing age of the poultry. Thus, the user can check the quality of the poultry or food stuff 20 in general at the beginning of the cooking process by using this ratio for instance.

As was shown by means of FIG 4 to 7 different components of the impedance Z of the food stuff 20 or parameters derived thereof can be used to determine the age or freshness of the food stuff, preferably before or at the beginning of the cooking process.

Accordingly, reference data for the characteristic functional relationship R(f) in FIG 4 or X(f) in FIG 5 or ϕ(f) in FIG 6 or ϕ(f1)/ϕ(f2) in FIG 7 for the various food stuff 20 of different age provided as cooking goods, in particular meat or animals to be cooked such as poultry or pork or veal or fish etc., can be empirically obtained by calibration measurements or calculated by interpolation or extrapolation. The reference data is stored in a storage or data base and used as reference data for comparison with actual data determined at the beginning or during a cooking process in order to determine the age of the poultry, meat or food stuff 20 in general which is going to be or is being cooked.

Instead of a ratio of the phase angles at two frequencies also a ratio of the capacitances at two frequencies could be used or of any other component of the impedance Z.

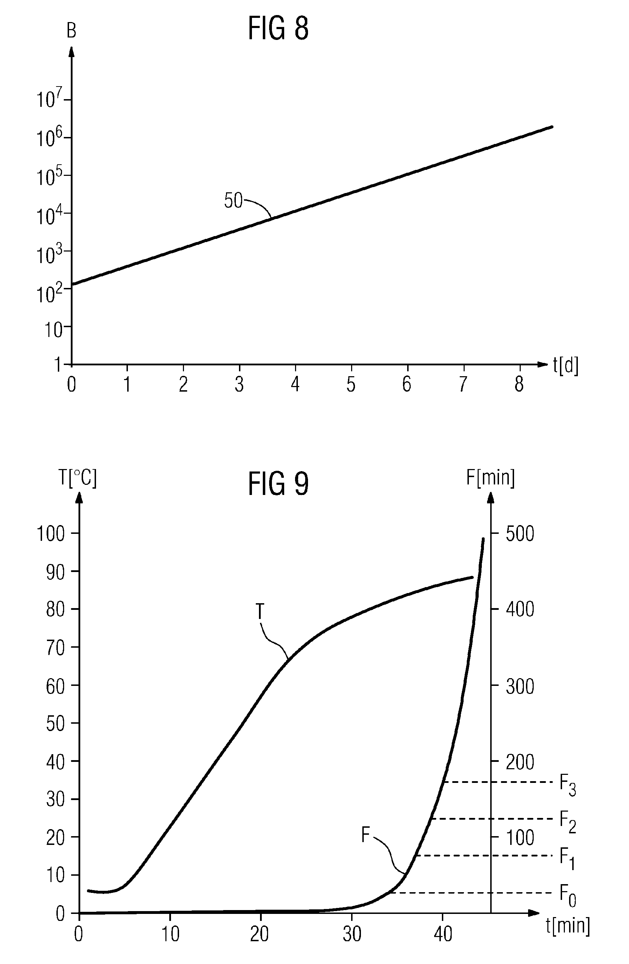

FIG 8 illustrates a schematic diagram of an amount B of bacteria as a function of the age t of the food stuff according to the preferred embodiment of the present invention. In this example, the shown bacterium is listeria monocytogenes. The food stuff is poultry. The temperature T is 5°C. It can be seen that the amount B of bacteria increases tenfold within two days. After eight days the amount B of bacteria increases by the factor of 10.000.

So from FIG 7 and the correlation between the ratio of phase angles ϕ at the frequencies f of 50 kHz and 5 kHz and the amount B of bacteria in FIG 8 at the same age t the user can determine not only the age of the poultry or food stuff in general but also estimate the amount B of bacteria.

The amount B of bacteria in the food stuff 20 gives information about the age or freshness of said food stuff 20 on the one hand and information about the progress of the cooking process of said food stuff 20 on the other hand. As shown in FIG 8, there is a correlation between the amount B of bacteria and the age t of the non-cooked food stuff 20. The amount B of bacteria increases within the non-cooked food stuff 20. However, when the food stuff 20 is cooked, then the amount B of bacteria is reduced. Thus, the cooking process causes a sterilization of the food stuff 20.

The sterilization of the food stuff 20 depends on the cooking temperature and on the length of time, at which the food stuff 20 has been exposed to the cooking temperature (actual cooking time). A sterilizing effect F is defined by:

$$F = D (\log N_0 - \log N) = r * D,$$

wherein D is a decay time, $N_0$ is the initial microbiological or microbial concentration in the food, N is the final microbiological concentration and r is the number of resulting decimal reductions. The decay time D represents the time period of exposing the food stuff 20 to a constant reference temperature $T_0$, which is necessary for the concentration of vital cells to be reduced by ten times. In other words, the decay time D is the time period required for the inactivation of 90% of the initially present cells or spores. For example, the reference temperature $T_0$ is chosen to be 71°C, which is used for pasteurisation.

A method as disclosed in EP 1 317 643 B9 which document is incorporated herein by reference can be applied to reduce the amount of bacteria and to provide sufficiently sterilized and cooked food stuff.

To this purpose, the data base comprises empirically pre-determined safety values $F_0$, $F_1$, $F_2$ ... $F_n$ for the sterilizing effect F with $F_0 < F_1 < F_2 < ... < F_n$. Said safety values $F_0$, $F_1$, $F_2$, ... , $F_n$ correspond to degrees of known bacterial content reduction or bacterial safety levels or status. For instance, if $F < F_0$, then the food stuff 20 is not consumable. If $F_0 < F < F_1$, then the food stuff 20 is consumable within five hours. If $F_1 < F < F_2$, then the food stuff 20 is consumable within one day. If $F_2 < F < F_3$, then the food stuff 20 is consumable within five days. Other or further values $F_i$ may be defined. Although one minimum safety value $F_0$ would suffice, more safety values $F_i$ help to improve the information for the user and the resolution and accuracy of the process.

The determined value of the sterilizing effect F is compared with the set of the predetermined safety values $F_0$, $F_1$, $F_2$ ... $F_n$ from the data base.

Different sets or vectors of safety values $F_i$ for i = 0, 1, 2, ...n should be provided for different types or categories of food as the food stuffs have different bacteriological growth or risks in particular according to HACCP standards. For instance meat or fish has a higher risk and growth of bacteria than vegetables. So different hazard groups for food types can be defined. Said data base, thus, contains the values $F_0$, $F_1$, $F_2$ ... $F_n$ associated to the kind or type of food and to the cooking time for said type of food.

Thus, it is advantageous to know the type of food that is being cooked in order to know the hazard group it is associated with or belongs to. The hazard group then allows for selection of the right set of safety values $F_i$ associated with this foodstuff and its hazard group. In other words, the food being cooked is classified according to pre-defined categories and a multiplicity or pre-defined safety values $F_i$ depending on the corresponding category is used which the actual value F is compared with and if the value F exceeds one of the values $F_i$ a signal or information is obtained indicating or containing the information on the safety level or status corresponding with the safety value, e.g. that the food is to be consumed by a corresponding date or time. Also, if a minimum safety level or reduction of bacteria has not been reached, because the temperature or cooking time was not sufficient, the control unit ca continue the cooking for a period sufficient to overcome this problem and to reach at least the minimum and first safety level or value $F_0$.

Now, the deriving of information on the food type of the food stuff using the detected impedance Z or its component according to the invention can be used to provide an automatic selection or recognition of the food type and thus its hazard group. The information about the food type as obtained from the detected impedance Z or its component, as described above, will be supplied from the evaluation unit to the control unit and be used to define the hazard group and thus the safety values Fi for the determination of the reduction of bacteria in the food stuff or the sterilizing effect F.

This means that it is not necessary any more that the user provides such information about the food type or its hazard group manually through selection means as described in EP 1 317 643 B9, although such manual input can still be provided. The automatic recognition of the food type according to the invention is thus a first possibility to modify or supplement the method as described in EP 1 317 643 B9.

The progress of the cooking process correlates with the content of bacteria within the food stuff 20. During the cooking process the sterilizing effect F can be determined by integrating the temperature T in a period of time:

$$F = {}_{t1}\!\int^{t2} 10^{(T-T_0)/\tau}\, dt = r * D,$$

wherein T is the temperature at the coldest point inside the food stuff 20 or the lowest temperature measured at the various check points, $T_0$ is the reference temperature, t1 is the instant in which a temperature exceed a predetermined value, t2 is the instant of the final detection and T is a temperature increment from the reference temperature To in order to obtain the decimal reduction. Thus, T is a characteristic constant of this kind or category or type of food stuff.

In order to obtain the sterilizing effect F during the cooking process, the temperature T within the food stuff 20 has to be determined. The reference temperature To and the temperature increment T are given constants.

Now, in a second possibility to modify or supplement the method as described in EP 1 317 643 B9 according to the invention the temperatures in the different sub-regions or at the different check points within the food stuff 20 are measured indirectly by evaluating the measurements of the electrical impedance Z or its electrical component of the food stuff in these sub regions or at check points according to the invention. The temperature T is calculated from the electrical impedance Z of the food stuff or its component.

In particular, the detected phase angle $\phi$ of electrical impedance Z of the food stuff 20, at two or more different frequencies f is calculated. Preferably, the electrical impedance Z is detected at the frequencies f of 50 kHz and 5 kHz. The ratio $\phi(f1)/\phi(f2)$ of the phase angles $\phi$ at two frequencies f1 and f2 allows the determination of the temperature T inside the food stuff 20 as described above already for instance referring to FIG 3.

Since the food probe 10 includes several pairs of electrodes 14 and 16, the temperature T is determined at several points inside the food stuff 20. The lowest value of the temperature T is selected, since the coldest point inside the food stuff 20 is relevant. Normally, the coldest point is the innermost portion inside the food stuff 20. However, if the food stuff 20 has a heterogeneous structure, then the coldest point can be out of the centre of said food stuff 20.

In an embodiment not shown, it is, however, also possible to provide the food probe with a plurality of temperature sensors in the vicinity of the impedance sensors constituted by the pairs of electrodes 14 and 16 and to measure the temperatures in the different sub-regions or at the different check points directly, for instance in a manner as described in EP 1 317 643 B9.

FIG 9 illustrates a schematic diagram of the temperature T and the sterilizing effect F of the food stuff 20 as a function of the time t according to the preferred embodiment of the invention. In this example, the food stuff 20 is chicken. The cooking process takes 45 minutes over all.

FIG 9 clarifies that the food stuff 20 is consumable after about 33 minutes, when the sterilizing effect F has reached the first safety value $F_0$. After 40 minutes the sterilizing effect F has reached the fourth safety value $F_3$. FIG 9 clarifies further that the sterilizing effect F has reached the safety values $F_0$, $F_1$, $F_2$ and $F_3$, in each case, after the temperature T has exceeded the reference temperature To of 71°C corresponding with the pasteurisation temperature.

The initial microbiological concentration No in FIG 9 is $10^4$ cells per gram. The final microbiological concentration N should be $10^2$ cells per gram, which is suitable for consumption within five hours. The reference microorganism is listeria monocytogenes, which has a decay time D of 0.23 minutes at the reference temperature To of 71°C. The temperature increment z is 10 °C.

A first safety value $F_0$ can be calculated:

$$F_0 = D \, (\log N_0 - \log N) = 0.23 \text{ min} * (\log 10^4 - \log 10^2)$$
$$= 0.46 \text{ min}$$

This means, that the required sanitizing or sterilising effect can be attained by a heat treatment process, which is effective as or corresponds to a permanent treatment for 0.46 minutes at a constant temperature of 71°C. The actual sterilizing effect F is calculated by:

$$F = {}_{t1}\int^{t2} 10^{(T-71°C)/10°C} \, dt.$$

During the heat treatment or cooking process of the food stuff, the value of the sterilizing effect F is determined in a continuous manner by integrating the temperature T in a period of time.

Now, one further problem remains that the initial microbiological or microbial concentration or contamination with microorganisms No in the food stuff 20 is not known *ab initio.* Rather, the initial concentration No has to be estimated as a possible maximum value which is allowed for the food at the point of sale or supply and the food suppliers by microbiological standards, such as for instance $10^4$ cells per gram as indicated in EP 1 317 643 B9. This means that, for these security reasons, the cooking time is usually unnecessarily long and the quality of the cooked food suffers, e.g. the food becomes too dry or cooked for too long.

Now, in a third possibility to modify or supplement the method as described in EP 1 317 643 B9 according to the invention the information on age or freshness of the food stuff derived from the detected impedance or component thereof is related to the initial microbial concentration No in the food stuff as shown in FIG 8 and described above.

The (approximate) age of the food stuff is derived according to the invention from the impedance Z or its component as described above.

Now, using a relation between the age t in days and the concentration of bacteria as shown in FIG 8 (or a similar empirical relation obtained previously) one can start from the maximum allowed microbial concentration at the point of sale which corresponds to a maximum age of the food under proper cooling or freezing storage conditions and calculate the real microbial concentration down to the real age determined, wherein the number r of resulting decimal reductions is diminished.

So, for instance in the example of FIG 8 if the maximum allowed microbial concentration could be assumed to be $10^4$ which would correspond to an age of the food stuff of approximately 3.75 days and the age of the food stuff determined from the impedance Z would be approximately 1.75 days the initial microbial concentration No in the food stuff could be estimated to be approximately $10^3$ instead of $10^4$ and the safety value F would be for No = $10^3$

$$F_0 = 0.23 \text{ min} * (\log 10^3 - \log 10^2) = 0.23 \text{ min}$$

instead of 0.46 min for No = $10^4$ meaning r = 1 instead of r =2 for the number r of resulting decimal reductions. This safety value $F_0$ diminished by half in its value results in a shorter cooking time with the same or sufficient sterilizing effect F.

The examples shown by FIG 1 to 9 have clarified that several coordinates or components of the electrical impedance Z provide substantial information about the food stuff.

Since the one or more food probes 10 include several check points, the information about the food stuff 20 is determined for several points inside the food stuff 20. Preferably, the most disadvantageous or hazardous information is used for monitoring and controlling the cooking process. For example, while determining the temperature T of the food stuff 20, the lowest value of the temperature T and the coldest point inside the food stuff 20 is relevant.

Further parameters like capacitance and specific dielectric constants can be calculated from the detected quantities.

Additionally, the first and/or second derivative of the detected and/or calculated quantities or parameters can be determined.

In order to obtain more information the temperature of the food stuff 20 can be detected additionally.

A teach-in function can be implemented. Such a teach-in function allows the user a possibility to train the cooking oven for recognizing individual recipes or food stuff, which are not yet in the data base.

The present invention can also be embedded in a computer program product, which comprises all the features enabling the implementation of the method described herein. Further, when loaded in a computer system, said computer program product is able to carry out these methods.

According to the preferred embodiment of the present invention the electrical parameters are coordinates of the electrical impedance in the complex plane. The complex plane can be represented by a number of coordinate systems. The electrical parameters are formed by the according coordinates. The present invention bases on the cognition, that there is a correlation between the coordinates of the electrical impedance in the complex plane and the properties of the food stuff.

For example, the electrical parameters are Cartesian coordinates of the electrical impedance in the complex plane. The Cartesian coordinates of the electrical impedance in the complex plane are the ohmic resistance and the reactance. In general, the ohmic resistance and the reactance of the food stuff have different relationships to the quality of the food stuff. Thus, the ohmic resistance and the reactance contain more information of the food stuff as one of these parameters.

According to a further example, the electrical parameters are polar coordinates of the electrical impedance in the complex plane. The polar coordinates of the electrical impedance in the complex plane are the modulus and the phase angle of the electrical impedance.

Further, the electrical parameters may be functions of at least one coordinate of the electrical impedance in the complex plane. Such functions of the coordinates of the electrical impedance are preferred, which show a significant dependence of the properties of the food stuff. For example, the trigonometric functions of the phase angle can be used.

According to another embodiment of the present invention, a ratio of phase angles of the electrical impedance at two different frequencies is calculated as a function of the temperature of the food stuff. This ratio of phase angles depends on the temperature of the food stuff, so that the temperature inside the food stuff can be determined.

Further, the first and/or second derivatives of the detected and/or calculated parameters may be determined.

In particular, the data base comprises frequency spectra of the electrical impedance and/or of the calculated electrical parameters. For example, the data base may comprise the frequency spectra of the phase angle of the electrical impedance for a plurality of types of food stuff.

According to a special embodiment of the present invention, the data base can be supplemented by the user. Thus, the user can adapt the data base to individual recipes. Such a teach-in function allows the user a possibility to train the cooking oven for recognizing individual recipes, which are not yet in the data base.

Additionally, further electrical parameters of the food stuff may be calculated from the electrical impedance. For example, the capacity of the food stuff is calculated from the electrical impedance.

**List of reference numerals**

| 10 | food probe |
|---|---|
| 12 | rod |
| 14 | first electrode |
| 16 | second electrode |
| 18 | spike |
| 20 | food stuff |
| 22 | electric field |
| 24 | frequency spectrum of phase angle for potatoes |
| 26 | frequency spectrum of phase angle for cauliflowers |
| 28 | frequency spectrum of phase angle for pork |
| 30 | frequency spectrum of phase angle for turkey |
| 32 | ratio of phase angle as function of temperature |
| 34 | point where all proteins are denaturated |
| 36 | curve of ohmic resistance of fresh poultry |
| 38 | curve of ohmic resistance of aged poultry |
| 40 | curve of reactance of fresh poultry |
| 42 | curve of reactance of aged poultry |
| 44 | curve of ohmic resistance of fresh poultry |
| 46 | curve of ohmic resistance of aged poultry |
| 48 | curve of ratio of phase angle as function of time |
| 50 | amount of bacteria |
| Z | electrical impedance |
| R | ohmic resistance |
| X | reactance |
| Mod(Z) | modulus of the electrical impedance Z |
| $\phi$ | phase angle of the electrical impedance Z |
| f | frequency |
| T | temperature |
| t | time |

| B | amount of bacteria |
|---|---|
| F | sterilizing effect |
| $F_n$ | safety values |
| D | decay time |
| No | initial microbiological concentration |
| N | final microbiological concentration |
| N | number of resulting decimal reductions |
| To | reference temperature To |
| t1 | instant of temperature exceeding a predetermined value |
| t2 | instant of final detection |
| z | temperature increment |

**Claims**

1. A method for controlling a cooking process of food stuff (20) comprising the steps of:

   a) detecting at least one physical property, in particular an electrical impedance (Z) or at least one component thereof and/or a temperature (T), at at least one check point or at a plurality of check points (22) in a, preferably inner, region of the food stuff (20),
   b) deriving from said at least one detected physical property, in particular impedance or component thereof and/or temperature, food type information about the type of food of the food stuff (20) and/or food age information about the age of the food stuff (20),
   c) deriving from said at least one detected physical property, in particular impedance or component thereof and/or temperature, a bacterial information (e.g. F or $F < F_0$) about the bacterial concentration in the food stuff (20) or about a reduction in the bacterial concentration in the food stuff (20),
   d) using the food type information of the food stuff (20) and/or the food age information of the food stuff (20) in said step of deriving the bacterial information (e.g. F or $F < F_0$),
   e) providing said bacterial information (e.g. F or $F < F_0$) to a user interface and/or to an information output unit and/or to a control unit for adapting the cooking time and/or cooking temperature dependent on this bacterial information.

2. Method according to claim 1, comprising at least one or an arbitrary combination of the following steps or features:

   a) the same physical property, in particular the impedance or component thereof or temperature, is detected for deriving the food type information and the age information and/or for deriving the food type information and the bacterial information and/or for deriving the food age information and the bacterial information,
   b) the physical property for deriving the food type information is the impedance or component thereof,
   c) the physical property for deriving the food age information is the impedance or component thereof,
   d) the physical property for deriving the bacterial information is the temperature and/or the impedance or component thereof,
   e) for deriving the food type information and the age information and/or for the food type information and the bacterial information and/or the food age information and the bacterial information the corresponding at least one physical property is detected at the same time and/or the same detected physical property values or signals are used.

3. The method according to claim 1 or claim 2, comprising at least one or an arbitrary combination of the following steps or features:

a) checking whether the bacterial information (F) derived during the cooking process has reached a minimum safety level ($F_0$) for sufficient reduction of bacterial concentration,

b) determining the minimum safety level ($F_0$) dependant on a value for the initial bacterial concentration ($N_0$), in particular by using a formula $F_0 = D (\log No - \log N)$ wherein D is a pre-given decay time, No is the initial bacterial concentration and N is the final maximum bacterial concentration to be reached,

c) if the bacterial information (F) derived during the cooking process has not reached the minimum safety level ($F_0$), the control unit adapts the cooking time and/or cooking temperature in continuation of the cooking process until the minimum safety level ($F_0$) is reached,

d) providing further safety levels $F_1, ... F_n$ for n > 1 corresponding to different degrees of bacterial concentration reduction or bacterial safety levels,

e) wherein in particular $F_0 < F_1 < F_2 < ... < F_n$ and/or wherein in particular said safety levels $F_0, F_1, F_2, ... , F_n$ correspond to degrees of bacterial concentration reduction or bacterial safety levels or status, for instance, if $F < F_0$, then the food stuff (20) is not consumable, and/or if $F_0 < F < F_1$, then the food stuff 20 is consumable within five hours and/or if $F_1 < F < F_2$, then the food stuff (20) is consumable within one day and/or if $F_2 < F < F_3$, then the food stuff (20) is consumable within five days.

f) if the bacterial information (F) derived during the cooking process has not reached the minimum safety level ($F_0$) or another safety level ($F_1, F_2 ... F_n$), if provided, then providing such bacterial information (F) to the user interface and/or to an information output unit in order to inform the user accordingly.

4. The method according to claim 3, comprising at least one or an arbitrary combination of the following steps or features:

a) using the food type information derived from said at least one detected physical property to determine the minimum safety level ($F_0$) dependent on the type of food of the food stuff

b) wherein in particular in a type of food where bacterial growth is larger the minimum safety level ($F_0$) is set to be higher and/or wherein in particular the types of food are classified in at least two different hazard groups,

c) using the food age information derived from said at least one detected physical property to determine the initial bacterial concentration ($N_0$) and/or the minimum safety level ($F_0$),

d) in particular by using the maximum allowed bacterial concentration at the point of sale which corresponds to a maximum age of the food under proper cooling or freezing storage conditions and calculating the actual initial bacterial concentration ($N_0$) and/or the minimum safety level ($F_0$) at the actual age of the food stuff obtained from the food age information.

5. The method according to one of the preceding claims, comprising at least one or an arbitrary combination of the following steps or features:

a) applying at an electric voltage (22) at each check point in the region of the food stuff (20) and measuring the impedance (Z) or the at least one component thereof as the physical property at each check point of the food stuff,

b) using at least one food probe (10) which is inserted into the food stuff (20),

c) the food probe(s) comprise(s) a number of electrode pairs (14, 16) for applying the electric voltage (22) which number preferably corresponds to the number of check points,

d) the food probe(s) comprise(s) at least one or a number of temperature sensor(s) for measuring the temperature at at least on check point which number of temperature sensors preferably corresponds to the number of check points,

e) the at least one electrical component of the electrical impedance (Z) is chosen from the group comprising the ohmic resistance (R), the reactance (X), the capacity, the inductivity, the modulus and the phase angle ($\phi$).

6. The method according to one of the preceding claims, comprising at least one or an arbitrary combination of the following steps or features:

a) deriving the food type information about the type of food of the food stuff, including in particular different types of meat and/or fish and/or vegetables, from said detected physical property (Z) before said step of deriving the food age information about the age of the food stuff (20) and/or deriving the food type information based on the same detected physical property (Z) values or signals as said step of deriving food age information,

b) using said food age information for adapting the cooking time and/or cooking temperature (T) of the cooking process dependent on this food age information, wherein in particular for less fresh or aged food stuff the cooking time is selected to be longer and/or the cooking temperature is selected to be higher or to be at a high level for a longer time.

7. The method according to one of the preceding claims, comprising at least one or an arbitrary combination of the following steps or features:

   a) said step of deriving the food age information from said detected physical property (Z) is performed by comparison with stored pre-determined reference data for age of different food types, including in particular different types of meat and/or fish,
   b) said step of deriving food type information from said detected physical property (Z) is performed by comparison with stored pre-determined reference data for different food types, including in particular different types of meat and/or fish and/or vegetables,
   c) wherein in particular the reference data can be supplemented by the user.

8. The method according to one of the preceding claims, comprising at least one or an arbitrary combination of the following steps or features:

   a) said step of detecting the physical property (Z) of the food stuff for a subsequent deriving of food age information of the food stuff (20) takes place before or at the beginning of the cooking process and/or in a predefined temperature range, preferably low, temperature e.g. room temperature,
   b) in said step of detecting the physical property (Z) of the food stuff (20) for a subsequent deriving of food type information or food age information of the food stuff (20) several values are measured in a pre-given time interval, e.g. several minutes, at pre-determined instants of time, e.g. every 10 to 60 seconds,

9. The method according to one of claims 1 to 8, comprising at least one or an arbitrary combination of the following steps or features:

   a) detecting the electrical impedance (Z) or the at least one component thereof at one, two or more frequencies (f), in particular of the electric fields (22) applied,
   b) one of the two different frequencies (f) is chosen to be 50 kHz and another one of the two different frequencies (f) is chosen to be 5 kHz,
   c) comparing the detected values of the impedance or component thereof at the one, two or more frequencies (f) with the same number of stored reference values for food types and/or age or freshness of food stuff previously determined at the same frequencies (f) and determining the food type or age with by determining the highest degree of coincidence with the reference values, e.g. by some mathematical norm, for instance Euclidian norm, or metric,
   d) using in said step of deriving food age information or food type information of the food stuff a function of the detected values of the impedance or component thereof at the one, two or more frequencies,
   e) using in said step of deriving of food age information or food type information of the food stuff a ratio of two detected values of the impedance (Z) or component thereof at two different frequencies (f),
   f) using in said step of deriving food age information or food type information of the food stuff (20) a difference or sum of two detected values of the impedance (Z) or component thereof at two different frequencies (f),
   g) using in said step of deriving food age information or food type information of the food stuff (20) a ratio of a difference and the sum of two detected values of the impedance (Z) or component thereof at two different frequencies (f),

10. The method according to one of claims 1 to 9, comprising at least one or an arbitrary combination of the following steps or features:

   a) using in said step of deriving food age information about the age of the food stuff (20) the ohmic resistance (R) as the component of the impedance,
   b) the ohmic resistance (R) over the same frequency spectrum for fresh food stuff differs from that of aged food stuff in absolute values as well as in the first derivative and the second derivative,
   c) at lower frequencies, the ohmic resistance (R) of fresh food stuff, in particular meat such as poultry, is higher than that of aged food stuff and/or wherein the ohmic resistance (R) of fresh food stuff decreases more steeply with increasing frequency than that of aged food stuff and/or wherein, at high frequencies (f), the ohmic resistance (R) of the fresh food stuff is higher than that of aged food stuff and/or wherein the curve of the ohmic resistance (R) for fresh food stuff has a turning point in contrast to aged food stuff, wherein
   d) the used value of the ohmic resistance (R) is selected from those values of the ohmic resistance (R) at the different check points, and/or
   e) the used value of the ohmic resistance (R) is the mean value calculated from those values of the ohmic resistance (R) at the different check points.

11. The method according to one of claims 1 to 10, comprising at least one or an arbitrary combination of the following steps or features:

    a) using in said step of deriving food age information about the age of the food stuff (20) the reactance (X) as the component of the impedance,
    b) the reactance over the same frequency spectrum for fresh food stuff differs from that of aged food stuff in absolute values as well as in the first derivative and the second derivative,
    c) the reactance of fresh food stuff, in particular meat such as poultry, as well as of aged food stuff has a minimum in a given frequency spectrum and the reactance (X) of the fresh food stuff at the minimum is smaller than the reactance (X) of the aged food stuff at the minimum, wherein
    d) the used value of the reactance (X) is selected from those values of the reactance (X) at the different check points, and/or
    e) the used value of the reactance (X) is the mean value calculated from those values of the reactance (X) at the different check points.

12. The method according to one of claims 1 to 11, comprising at least one or an arbitrary combination of the following steps or features:

    a) using in said step of deriving food age information about the age of the food stuff (20) the phase angle ($\phi$) as the component of the impedance,
    b) the phase angle ($\phi$) over the same frequency spectrum for fresh food stuff differs from that of aged food stuff in absolute values as well as in the first derivative and the second derivative,
    c) the phase angle ($\phi$) of fresh food stuff, in particular meat such as poultry, has a minimum in a given frequency spectrum whereas the phase angle ($\phi$) of aged food stuff has no minimum in this given frequency spectrum,
    d) the ratio of phase angles ($\phi$) at two different frequencies (f) decreases with increasing age of the food stuff and is in particular used to check the quality of the food stuff before or at the beginning of the cooking process, wherein
    d) the used value of the phase angle ($\phi$) and/or the ratio of phase angles ($\phi$) is selected from those values of the phase angles ($\phi$) and/or the ratio of phase angles ($\phi$), respectively, at the different check points, and/or
    e) the used value of the phase angle ($\phi$) and/or the ratio of phase angles ($\phi$) is the mean value calculated from those values of the phase angle ($\phi$) and/or the ratio of phase angles ($\phi$), respectively, at the different check points.

13. The method according to one of claims 1 to 12, comprising at least one or an arbitrary combination of the following steps or features:

    a) deriving from said detected impedance or component thereof information about the age or freshness of the food stuff by calculating one or more electrical parameters (R; X; $\phi$) of the food stuff (20) from the electrical impedance (Z) or component thereof and comparing said electrical parameters (R; X; $\phi$) with a data base,
    b) detecting, in another step during the cooking process and/or when the food stuff has been subjected to cooking already, the electrical impedance (Z) or the at least one component thereof of the food stuff again for determining the cooking progress and/or cooking temperature in the region of the food stuff,
    c) wherein in particular a ratio of phase angles ($\phi$) at two different frequencies (f) is calculated as a function of the temperature (T) of the food stuff (20),
    d) deriving further to or as the information about the age or freshness of the food stuff an information on the estimated amount or concentration of bacteria in the food stuff (20), wherein
    e) the used value of the ratio of phase angles ($\phi$) corresponds with the temperature (T) at the coldest check point of the food stuff (20).

14. A food probe (10) for inserting into food stuff (20), being provided for use in the method according to any one of the claims 1 to 13 and comprising:

    - at least one probe body, in particular an elongated rod (12), in particular made of a non-conductive material,
    - wherein at least a probing portion of the probe body (12) is provided for inserting into the food stuff (20),
    - at least one pair of electrodes (14, 16) arranged at the probing portion of the probe body (12), in particular a plurality of pairs of electrodes (14, 16), preferably arranged serially or one after the other along a longitudinal axis of the probe body (12),

- wherein each pair of electrodes (14, 16) comprises a first electrode (14) and a second electrode (16) arranged in a predetermined distance from each other, and is connected or connectable to a voltage supply for applying a voltage between the first electrode (14) and the corresponding second electrode (16) and to a control unit of a cooking oven or cooking appliance for measurement of the physical property, in particular impedance (Z) or component thereof, of the food stuff (20) arranged in between the electrodes (14, 16)
- wherein for at least one, preferably each, check point in the food stuff (20) at least a pair of electrodes is provided.

15. A food probe according to claim 14, wherein each food probe (10) further comprising at least one temperature sensor arranged at the probe body (12), wherein for or at least one, preferably each, check point in the food stuff (20) at least one temperature sensor is provided for measuring the temperature at that check point.

FIG 1

FIG 2

FIG 3

FIG 4

FIG 5

FIG 6

ϕ [°]

46

44

f

FIG 7

$\dfrac{\phi\ (50\ \text{kHz})}{\phi\ (5\ \text{kHz})}$

48

t [d]

0    1    2    3    4    5    6

# FIG 8

# FIG 9

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 11 15 8729

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/23093 A1 (ELECTROLUX PROFESSIONAL SPA [IT]; TASSAN MANGINA FRANCO [IT]) 21 March 2002 (2002-03-21) | 1,3-7,9, 13 | INV. G01N33/12 F24C15/00 |
| Y | * abstract; claims; figures * | 2,10-12, 15 | A23L1/01 |
| X | WO 2008/147988 A1 (SINGER MICHAEAL G [US]) 4 December 2008 (2008-12-04) | 14 | |
| Y | * claims * | 2,10-12, 15 | |
| A | WO 2004/106899 A1 (JACKMAN NICHOLAS [IE]; GRATTAN KENNETH TERENCE VICTOR [GB]; LEWIS ELFE) 9 December 2004 (2004-12-09) * the whole document * | 1,2,5-7, 9-15 | |
| A | US 2010/086655 A1 (SINGER MICHAEAL G [US]) 8 April 2010 (2010-04-08) * the whole document * | 1,2,5-7, 9-15 | |

TECHNICAL FIELDS
SEARCHED      (IPC)

G01N
F24C
A23L

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 2 January 2012 | Kraus, Leonie |

EPO FORM 1503 03.82 (P04E07)

Europäisches
Patentamt

European
Patent Office

Office européen
des brevets

**INCOMPLETE SEARCH
SHEET C**

Claim(s) completely searchable:
        1-9, 13-15

Claim(s) searched incompletely:
        10-12

Reason for the limitation of the search:

Features b) and c) of claim 10 relate to intrinsic properties of the
foodstuff and do not contain a technical disclosure to-be-carried-out.
The same applies to features b) and c) of claim 11 and to features b) and
c) of claim 12. Also the first part of claim 12 first-d) relates to
intrinsic food properties, while the rest of the claim is purely optional
("in particular").
Therefore a meaningful search is not possible here.

Europäisches
Patentamt

European
Patent Office

Office européen
des brevets

Application Number

EP 11 15 8729

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1(completely); 2, 13(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

Application Number

EP 11 15 8729

The Search Division considers that the present European patent application does not comply with the requirements of unity of invention and relates to several inventions or groups of inventions, namely:

1. claims: 1(completely); 2, 13(partially)

    claims 1, 2a, 2d, 13d (control a cooking process based on bacterial information)

    ---

2. claim: 2(partially)

    claims 2b, 2c (choose impedance as parameter to be measured)

    ---

3. claims: 2, 6(all partially)

    claims 2e, 6a (choose in which order to determine food properties)

    ---

4. claims: 3, 4

    claim 4 (judge a foodstuff as safe or unsafe)

    ---

5. claim: 5(partially)

    claims 5a, 5e (use a voltage to detect the impedance component)

    ---

6. claim: 5(partially)

    claims 5b,5c,5d,14,15 (use a probe which is inserted in the foodstuff)

    ---

7. claim: 6(partially)

    claim 6b (how to use the food age information -> adapt cooking time/temperature)

    ---

8. claims: 7, 9, 13(all partially)

    claims 7a, 7b, 9c, 13a (mathematical implementation -> compare with stored values)

    ---

9. claims: 8, 13(all partially)

    claims 8a, 13b (when to determine food property -> before/during cooking)

    ---

Europäisches
Patentamt

European
Patent Office

Office européen
des brevets

**LACK OF UNITY OF INVENTION
SHEET B**

Application Number

EP 11 15 8729

The Search Division considers that the present European patent application does not comply with the
requirements of unity of invention and relates to several inventions or groups of inventions, namely:

```
10. claim: 8(partially)

        claim 8b (plurality of measurements in a certain time
        interval)
                        ---

11. claim: 9(partially)

        claims 9a,9b,9d-g,13c,13e (measurements at a plurality of
        frequencies)
                        ---

12. claims: 10-12

        claims 10, 11, 12 (select impedance value)
                        ---
```

**ANNEX TO THE EUROPEAN SEARCH REPORT
ON EUROPEAN PATENT APPLICATION NO.**          EP 11 15 8729

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2012

| Patent document cited in search report | | Publication date | Patent family member(s) | | Publication date |
|---|---|---|---|---|---|
| WO 0223093 | A1 | 21-03-2002 | AT | 354768 T | 15-03-2007 |
| | | | DE | 60126789 T2 | 31-10-2007 |
| | | | EP | 1317643 A1 | 11-06-2003 |
| | | | ES | 2282289 T3 | 16-10-2007 |
| | | | IT | PN20000054 A1 | 13-03-2002 |
| | | | US | 2004020916 A1 | 05-02-2004 |
| | | | WO | 0223093 A1 | 21-03-2002 |
| WO 2008147988 | A1 | 04-12-2008 | CN | 101680866 A | 24-03-2010 |
| | | | WO | 2008147988 A1 | 04-12-2008 |
| WO 2004106899 | A1 | 09-12-2004 | NONE | | |
| US 2010086655 | A1 | 08-04-2010 | NONE | | |

EPO FORM P0459

For more details about this annex : see Official Journal of the European Patent Office, No. 12/82

**EP 2 500 725 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060174775 A1
- DE 3621999 A1
- EP 1317643 B9